# FASCICULE DE BREVET EUROPEEN

(11) **EP 1 164 921 B1**
(45) Date de publication et mention de la délivrance du brevet: **20.11.2002**
(21) Numéro de dépôt: 00915271.1
(22) Date de dépôt: 31.03.2000
(51) Int. Cl.: A61B 3/14

(54) **DISPOSITIF D'OBSERVATION D'UN CORPS A HAUTE RESOLUTION**
EINRICHTUNG ZUR BEOBACHTUNG EINES KÖRPERS MIT HOHER AUFLÖSUNG
HIGH RESOLUTION DEVICE FOR OBSERVING A BODY

(30) Priorité: 01.04.1999 FR 9904086
(43) Date de publication de la demande: 02.01.2002
(73) Titulaire: UNIVERSITE PARIS 7 - Denis DIDEROT, F-75005 Paris (FR)
(72) Inventeur: LE GARGASSON, Jean-François, 94350 Villiers s/Marne (FR); LENA, Pierre, 75013 Paris (FR); BOCCARA, Claude, 75012 Paris (FR); DUBOIS, Arnaud, 91940 Les Ulis (FR)
(74) Mandataire: Warcoin, Jacques
(86) Numéro de dépôt international: FR0000823
(87) Numéro de publication internationale: WO00059368

(56) Documents cités:
- WO-A-98/27863
- DE-A- 4 222 395
- US-A- 5 615 278
- LIANG J ET AL: "OBJECTIVE MEASUREMENT OF WAVE ABERRATIONS OF THE HUMAN EYE WITH THE USE OF A HARTMANN-SHACK WAVE-FRONT SENSOR" JOURNAL OF THE OPTICAL SOCIETY OF AMERICA - A,US,OPTICAL SOCIETY OF AMERICA, WASHINGTON, vol. 11, no. 7, juillet 1994 (1994-07), page 1949-1957 XP002064186 ISSN: 0740-3232

## Description

L'invention concerne les dispositifs d'observation de corps, par exemple de corps vivants ou d'objets manufacturés.

Elle concerne notamment les dispositifs d'observation de l'oeil.

On connaît de nombreux dispositifs d'observation de l'oeil tels que biomicroscopes, ophtalmoscopes ou rétinographes. Ils servent à différents types d'examens tels qu'observations anatomiques directes ou explorations par le truchement de colorants tels que la fluorescéine, le vert d'indocyanine, le rose bingal, ou l'orange d'acridine.

Avec certains appareils expérimentaux récents, les explorations fonctionnelles sont également possibles comme l'étude des fixations ou la projection de tests destinés à l'enregistrement des activités électriques des voies visuelles. Dans ce cas, une image est directement formée sur la rétine à l'aide d'une modulation d'un faisceau d'illumination.

Les appareils les plus classiques sont basés sur l'éclairement global d'un champ de 5° à 60° en lumière poly- ou monochromatique.

Ces appareils nécessitent généralement de séparer les pupilles d'illumination et d'observation selon la règle dite de Gullstrand.

Les rétinographes utilisent une surface sensible photographique. Ils nécessitent des niveaux d'éclairement rétiniens beaucoup plus élevés que ceux requis pour l'observation ophtalmoscopique directement par la rétine de l'observateur. Leur niveau de performance est essentiellement défini par la résolution optique du système.

Plus récemment sont apparues des surfaces sensibles permettant des enregistrements vidéoscopiques à des longueurs d'ondes visibles ou non visibles par l'oeil humain. La résolution de ces appareils est alors soumise, en plus des limitations optiques, aux limitations de l'acquisition électronique.

Dans le domaine de l'exploration de la cornée et du cristallin, on connaît les systèmes d'illumination dont la source d'illumination a la forme d'un cercle ou d'une fente à largeur variable, et dont l'image optique est focalisée sur le plan d'observation.

Dans de tels dispositifs, on adopte un système d'observation constitué par un système monoculaire ou binoculaire grossissant solidaire du dispositif d'illumination. L'image des tissus peut être observée soit directement soit par l'intermédiaire d'une lentille additionnelle située près de l'oeil. Ces appareils répondent aux dispositions d'une règle de séparation des pupilles appelée règle de Gullstrand.

Plus récemment ont été décrits des appareils de microscopie cornéenne et/ou cristallinienne. Ces appareils sont très proches de ceux de la microscopie conventionnelle. Ces appareils utilisent le fait que les milieux antérieurs de l'oeil permettent de travailler avec des angles d'ouverture très larges, selon la technique dite en immersion.

Ces dernières années une nouvelle technique de microscopie dite confocale a également été proposée pour la visualisation de la cornée. La technique confocale présente l'intérêt de sélectionner un plan de coupe optique ayant une certaine épaisseur en Z. Les appareils faisant appel à cette technique ont donc des propriétés tomographiques, c'est à dire qu'ils permettent d'isoler un plan d'observation dans un milieu diffusant. Des techniques vidéoscopiques permettent d'acquérir en temps réel une grande série de coupes optiques dans des plans successifs.

Dans le domaine de l'exploration rétinienne on a également proposé une technique basée sur des principes différents de principes précédemment définis par Helmholtz au milieu du dix neuvième siècle.

Le brevet US 4 213 678 de POMERANTZEFF et WEBB a ainsi proposé un éclairement d'une petite surface balayant le champ d'exploration. Ce brevet enseigne l'usage d'un balayage optomécanique permettant de réaliser la déviation à deux dimensions d'un faisceau lumineux de diamètre inférieur à celui de la pupille de l'oeil. L'appareil collecte à pleine pupille le flux réfléchi et/ou diffusé par les tissus oculaires. Cet appareil respecte la règle de Gullstrand, ce qui limite la résolution par l'utilisation d'une pupille d'illumination réduite. Ce document propose de remédier à la présence de reflets en utilisant la lumière polarisée.

COHEN SABBAN, ROUSSEL & SIMON proposent dans le brevet EP 145 563 de faire emprunter au flux réfléchi et/ou diffusé par les tissus oculaires le même trajet que le flux d'illumination. Ce flux réfléchi suit la voie de déviation optique et devient ainsi immobile. Les auteurs nomment cette action stabilisation du faisceau. Ce dispositif optique autorise la filtration du faisceau de retour issu de l'oeil, en disposant des éléments de filtration spatiale dans un plan conjugué de la source et des tissus oculaires observés. Cette description correspond à l'utilisation d'un dispositif confocal. Ce dispositif optique permet d'éliminer les reflets sans utiliser la règle de séparation des pupilles de Gullstrand.

L'utilisation d'une même pupille pour les voies d'illumination et d'observation procure une résolution supérieure à celle du dispositif du document US 4 213 678. L'utilisation de la filtration confocale permet d'augmenter le contraste en éliminant les flux diffusés par les plans sus et sous-jacents de celui observé.

En 1987, WEBB et HUGUES, dans le brevet EP 223 356 reprennent le principe de la stabilisation du faisceau d'illumination et d'observation, mais ils conservent la séparation des pupilles au niveau du faisceau de retour stabilisé. Ce dispositif est plus efficace que le précédent pour ce qui concerne l'élimination des reflets pupillaires, mais il présente toujours le défaut d'une limitation du flux collecté.

L'utilisation de dispositifs de filtration spatiale sur la voie de retour stabilisée permet d'obtenir des images dont la profondeur de champ est définie par le diamètre de la pupille de filtration. Cet aspect tomographique autorise la réalisation d'images en trois dimensions des tissus étudiés. Mais la résolution obtenue est liée aux aberrations géométriques et aux fluctuations des milieux transparents. Elle reste limitée à 30 µm sur la surface XY de la rétine et à 300 µm pour la profondeur Z du tissu rétinien.

Dans le domaine de l'exploration cornéenne et rétinienne, Izaat et al ont proposé une exploration par interférométrie avec un dispositif de type interféromètre de Michelson.

Ce dernier utilise une source d'illumination de faible cohérence spatio-temporelle. La résolution en profondeur dite Z est conditionnée par les caractéristiques de cohérence de la source. L'utilisation d'une diode électroluminescente autorise une résolution en Z de 15 µm dans l'oeil et de 20 µm au niveau de la cornée.

L'image d'une coupe optique est obtenue, en profondeur rétinienne Z, par une succession d'interférences réalisées entre le flux issu des tissus à analyser et un flux de référence venant d'un miroir situé dans la branche dite de référence. Chaque position du miroir de référence fournit un système d'interférences qui sera analysé et fournira les informations d'un pixel en Z.

L'exploration selon une ligne est obtenue grâce à un balayage optomécanique permettant d'explorer une nouvelle position, lorsque l'exploration en Z pour une position précédente est terminée. La résolution XYZ est de l'ordre de 20 µm dans les meilleures conditions.

Ce dispositif est sensible aux déplacements de l'oeil, car le temps d'acquisition est relativement long par rapport aux bandes passantes des mouvements oculaires. Des algorithmes de correction ne permettent de compenser que partiellement ce défaut. Ce dispositif donne une image de différentes positions relatives et ne permet pas de donner une image topographique fidèle et absolue. La résolution réelle obtenue est de l'ordre de 100 µm en X ou Y et 50 µm en Z.

Dans le domaine de l'optique adaptative, on a proposé dans le brevet US 5 777 719 d'insérer un dispositif de compensation de front d'onde utilisant un miroir déformable dans un système rétinographique conventionnel. Un point de la surface est éclairé par une diode super radiante qui permet de mesurer les déformations du front d'onde et de calculer la compensation qui doit être apportée par le miroir déformable. Cette compensation est réalisée sur une image complète du fond d'oeil, et acquise par une caméra CCD. Cette technique perrnet d'obtenir une résolution XY de 2µm, ce qui est insatisfaisant.

De plus, ce dispositif ne permet pas d'extraire un signal utile correspondant à un plan optique choisi du tissu étudié en évitant l'ensemble des réflexions et rétrodiffusions issues de plans sous- et sus-jacents.

Un but général de l'invention est de proposer un dispositif d'observation d'un corps, tel que le corps d'un être vivant ou tel qu'un objet manufacturé, présentant une résolution d'image particulièrement améliorée par rapport à celles fournies par les dispositifs existants.

Ce but est atteint selon l'invention grâce à un dispositif d'observation d'un corps, notamment d'un oeil, selon la revendication 1.

D'autres caractéristiques, buts et avantages de l'invention apparaîtront à la lecture de la description détaillée qui va suivre faite en référence aux figures annexées sur lesquelles :
- la figure 1 est un schéma sous forme de blocs fonctionnels d'un l'ophtalmoscope selon l'invention,
- la figure 2 est une représentation fonctionnelle détaillée d'un ophtalmoscope conforme à une première variante de l'invention, utilisant une technique de filtration confocale pour extraire des signaux issus de l'oeil ;

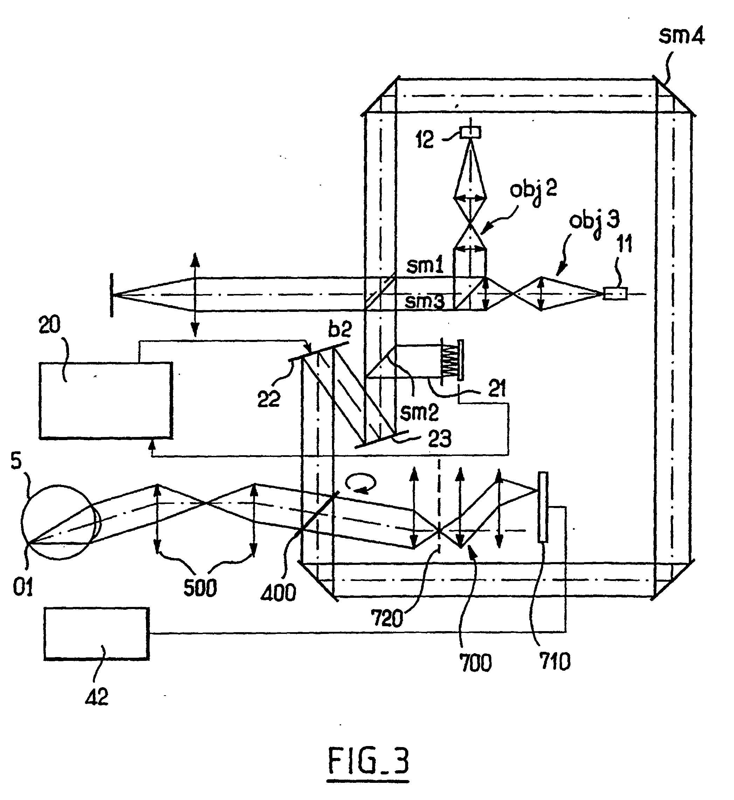

- La figure 3 est. une représentation fonctionnelle détaillée d'un ophtalmoscope conforme à une seconde variante de l'invention, utilisant une technique d'interférométrie pour extraire le signal issu de l'oeil ;

Dans la description qui va suivre, on appelle surface ou microsurface isoplanétique une surface d'observation de forme sensiblement plane, qui remplit la condition que les fronts d'onde issus d'un point quelconque tui appartenant sont identiques à une fraction de longueur d'onde près. Une telle surface peut par exemple être constituée matériellement par une face d'un corps ou peut également être constituée par un plan de coupe dans un milieu diffusant.

On entend par fraction de longueur d'onde un écart inférieur à une longueur d'onde.

On a proposé, dans le document « Objective measurement of wave aberrations of the human eye with the use of a Hartman-Schack wave front sensor », Liang V et al., journal of the optical society of America, vol.11, n°7, juillet 1994, une méthode de mesure de distorsions de front d'onde qui peut être utilisée dans le cadre de l'invention pour identifier la déformation de front d'onde et ainsi l'identité de forme ou non entre deux déformations de front d'onde. Cette méthode de mesure, qui y est exposée en détail dans le cas d'un faisceau sortant de l'oeil humain, est applicable à la détermination de la distorsion de front d'onde en sortie de tout corps observé.

Plus précisément, dans les dispositifs qui seront décrits par la suite, on définira le critère d'isoplanétisme d'une microsurface par le fait que les fronts d'onde émanant de tout point de cette surface sont identiques à environ un quart de longueur d'onde près.

Les microsurfaces isoplanétiques sont déterminées par une étude préalable des déformations du front d'onde sur une surface déterminée. Lorsque les déformations du front d'onde sortent de ce critère, c'est que la surface explorée n'est pas isoplanétique. Il convient alors conformément à l'invention de réduire celle-ci jusqu'à ce qu'elle le redevienne.

Ce procédé itératif d'identification d'une surface isoplanétique peut être réalisé au stade de la définition des-paramètres du dispositif de l'invention de manière à définir une étendue moyenne des surfaces isoplanétiques, ou être réalisé par un module automatique intégré à un dispositif selon l'invention, dont le rôle est, pour chaque emplacement choisi d'une surface, de déterminer son étendue pour qu'elle soit isoplanétique.

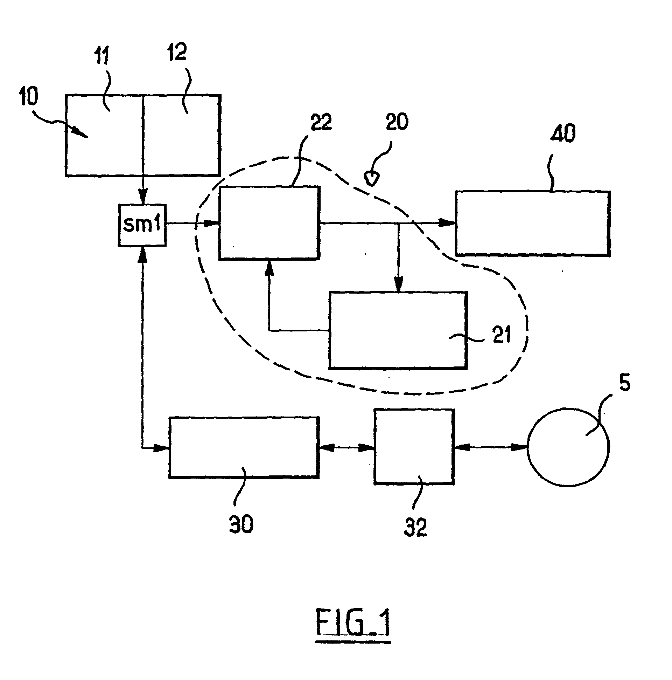

La structure de la figure 1 comprend quatre organes principaux, que sont un bloc source lumineuse 10, un dispositif d'optique asservie 20, un dispositif de balayage pas à pas 30 et un dispositif de captation et de réalisation d'image 40.

De manière préférentielle, le bloc source 10 comprend deux sources particulières une source 11 destinée à la mesure des déformations de front d'onde des surfaces isoplanétiques, et une source 12 destinée à la réalisation d'une image d'une surface isoplanétique.

L'intensité des sources 11 et 12 est choisie compatible avec les règles de sécurité concernant les tissus biologiques (Norme AFNOR C 43 801, 1992 et ANSI Z136.1, 1993). Les sources peuvent être modulées par une électronique de commande afin de n'utiliser que la source de mesure de front d'onde 11 lors d'une phase de mesure de front d'onde, et la source 12 seule lors d'une phase distincte de réalisation de l'image.

Le dispositif d'optique asservie 20 est placé sur le circuit du flux lumineux rétro-diffusé par le tissu oculaire 5. Il comprend un dispositif de mesure des déformations de front d'onde 21 et un dispositif de compensation ou correction de front d'onde 22.

Le dispositif de compensation 22 est situé avant le dispositif de mesure 21, sur le trajet du flux issu de l'oeil (ou d'un autre corps, tel qu'un objet manufacturé). Il constitue avec celui-ci un système asservi en boucle de réaction.

Le dispositif 21 est par exemple constitué par un système de mesure de front d'onde de type Hartmann & Shack divisant le faisceau en un grand nombre de sous-pupilles, ou encore par un dispositif d'analyse de courbure de front d'onde. Ce dispositif est préférentiellement situé dans un plan conjugué optique de la pupille du sujet, mais peut être placé dans tout autre plan jugé optimal. Ce système optique mesure les aberrations, notamment géométriques, de dioptres. et les fluctuations temporo-spatiales des indices des milieux transparents, ainsi que les aberrations engendrées par un dispositif utilisant des systèmes optiques de grande ouverture.

Le dispositif 22 peut être constitué par un miroir déformable comportant un certain nombre d'actuateurs susceptibles de le déformer. Il compense les différences spatio-temporelles de trajet optique qui constituent la déformation du front d'onde. Il est également prévu d'utiliser tout dispositif équivalent, tel qu'un ensemble de micro-miroirs indépendants ou des cristaux liquides. Ce dispositif compense ainsi les défauts optiques provenant de l'objet observé ou du système d'observation lui-même.

Le miroir 22, ici représenté comme réalisant une déviation du flux lumineux de 90°, est avantageusement placé de manière à réfléchir le flux selon un angle inférieur à 90° par rapport au flux arrivant sur le miroir.

Dans de telles conditions de boucle d'asservissement, la résolution est conditionnée à la fois par la limite de diffraction et par la qualité de ce système d'asservissement adaptatif.

Le dispositif de balayage pas à pas 30 a pour rôle de changer la microsurface observée. Il dirige les flux optiques émis par la source 12 de façon à explorer successivement une série de micro-surfaces. Pour cela il comporte par exemple des déviateurs mécano-optiques commandés par des moteurs pas à pas.

Le pas est déterminé par l'étendue des surfaces isoplanétiques que l'on désire explorer. Il peut être fixe ou être réglé par l'utilisateur selon la qualité de l'image et le champ qu'il veut observer. Pour l'observation des champs importants, dont la surface toute entière ne satisfait pas aux conditions d'isoplanétisme définies précédemment, on choisit le pas de sorte que les parties explorées successivement répondent aux caractéristiques d'isoplanétisme.

Dans un mode de réalisation préféré, le flux d'illumination et le flux rétrodiffusé par les tissus empruntent le même trajet, de sorte que le faisceau de retour est stabilisé par le dispositif de déviation 30.

Un séparateur SM1 est placé en liaison optique entre les trois modules que sont le bloc source 10, le module d'optique asservie 20 et le module de balayage 30. Ce séparateur SM1 a pour rôle de transmettre le flux émis par les sources 11 et 12 au dispositif de balayage 30 et de transmettre le flux de retour venant du module de balayage 30 au dispositif d'optique asservie 20. La partie d'optique adaptative 20 reçoit alors un signal stabilisé.

Le dispositif de captation et de réalisation d'image 40 est un dispositif de réception optoélectronique comprenant un bloc électronique et informatique muni d'un bloc horloge qui génère la séquence de tous les événements, amplifie et traite tous les signaux issus de capteurs et d'éventuels éléments de modulation du flux d'illumination ou d'éventuels éléments de commande de filtration dynamique qui seront décrits dans la suite. Il réalise en outre le traitement des informations pour la visualisation et la mémorisation des images obtenues.

Les dispositifs décrits par la suite reprendront les éléments du dispositif de la figure 1, ces éléments communs portant les mêmes références.

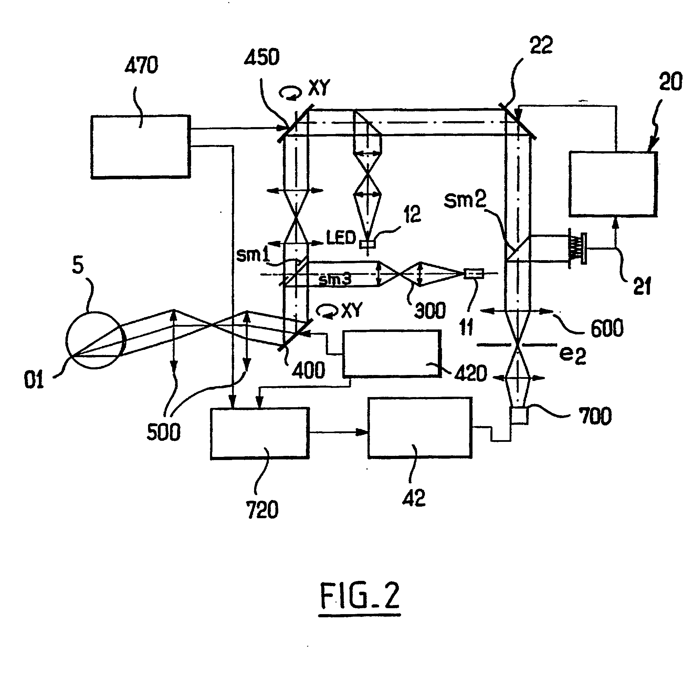

On décrira maintenant plus précisément le dispositif de la figure 2.

La mesure du front d'onde est effectuée dans ce dispositif par la formation d'un point d'illumination le plus petit possible dans le tissu exploré. Pour cela, l'émission lumineuse d'une diode super radiante 11 arrive à l'oeil 5 après avoir traversé une lentille 300, être réfléchie sur un miroir séparateur SM1, puis sur un miroir mobile de changement de microsurface 400 et avoir traversé un couple de lentilles 500 formant également l'entrée optique du dispositif.

L'analyseur de front d'onde 21 est situé dans un plan optique conjugué de celui de la pupille du sujet. Pour parvenir à cet analyseur 21, le flux rétrodiffusé franchit le couple de lentilles 500, est réfléchi par le miroir mobile 400, traverse le miroir séparateur SM1 et est réfléchi par un second miroir mobile 450.

La source 12 d'illumination est placée directement en amont de ce miroir 450, le flux émis par la source 12 étant dirigé sur le miroir mobile 450 par un miroir semi-transparent à 45°.

L'ensemble optique est prévu pour que le flux d'illumination émis par la diode 12 forme sur la rétine un point de concentration de lumière. Comme on le décrira par la suite, seuls les rayons rétrodiffusés par ce point de concentration seront prélevés. Le dispositif utilise donc la technique confocale à balayage.

Le miroir 450 est ainsi adapté à balayer l'intérieur de la microsurface correspondant à la position choisie du miroir 400. L'ensemble de la surface isoplanétique correspondant à chaque position du miroir 400 est balayé par le miroir 450.

La diode 11 a pour rôle d'éclairer un point de la rétine de l'oeil 5, de sorte que le dispositif de compensation 20 déduise par calcul sur le flux rétrodiffusé par ce point une correction de front d'onde à appliquer aux signaux optiques le traversant.

Ainsi, pour chaque calcul de correction, c'est à dire pour chaque point éclairé par la diode 11, une nouvelle compensation est appliquée à l'ensemble des flux lumineux prélevés sur l'oeil 5, c'est à dire aux flux lumineux provenant de la diode 12, formant un point de concentration lumineuse sur celui-ci, rétrodiffusés par les tissus, et rejoignant directement l'entrée optique 500 du système depuis le point de concentration des rayons lumineux.

Selon l'invention, à chaque nouvelle position du miroir 400 est associé un nouveau calcul de compensation et une nouvelle correction par le miroir 22. L'étendue de la microsurface balayée par le miroir 450 pour une position donnée du miroir 400 étant choisie pour que cette surface soit isoplanétique, la correction appliquée par le module 20 reste constante pendant le balayage de cette surface. L'ensemble des flux prélevés sur cette surface est donc soumis à cette correction calculée préalablement, et qui est adaptée à cette surface choisie du fait qu'elle présente le caractère d'isoplanétisme. Lorsque la surface isoplanétique est changée, c'est à dire lorsque le miroir 400 passe à une nouvelle position, un nouveau calcul de compensation est réalisé à partir de l'illumination par la diode 11 d'un point de cette nouvelle surface isoplanétique.

Les moyens de prélèvement de la lumière, c'est à dire notamment les miroirs 400 et 450 et le dispositif de captation 400, et les moyens de prélèvement, c'est à dire notamment le module de compensation 20 et le miroir 400, sont donc synchronisés de sorte qu'un nouveau point de calcul de compensation est utilisé de manière optimale par les déviateurs 400 et 450 qui exploitent cette correction pour l'ensemble d'une étendue de prélèvement adaptée à cette correction. On tire ainsi un bénéfice maximal de chaque nouvelle correction. Pour illuminer un point de mesure de déformation de front d'onde situé dans la surface de prélèvement, le flux émis par la diode 11 est dirigé sur le miroir 400 par l'intermédiaire d'un miroir semi-transparent SM1, parallèlement à une direction des rayons d'illumination correspondant à une position moyenne du miroir 450.

Le miroir 400 est commandé par un module 420 synchronisé à un capteur 700 par un module de synchronisation 720. Le miroir 450 est lui aussi commandé par un module 470 synchronisé au miroir 400 et au capteur 700 par l'intermédiaire du module 720.

Selon la variante de l'invention décrite ici, l'étendue des surfaces isoplanétiques est déterminée à l'avance. Cependant, on prévoit selon l'invention de munir le dispositif d'un module apte, pour chaque nouvelle position du miroir 400, à déterminer l'étendue de la surface de prélèvement correspondant à cette position de sorte que celle-ci vérifie le critère d'isoplanétisme.

La micro-surface isoplanétique étudiée O1 est illuminée point après point par la diode électroluminescente 12 focalisée sur le plan tissulaire étudié. Le point d'illumination est du diamètre le plus faible possible.

Le dispositif de balayage supplémentaire 450 est situé dans un plan conjugué optique de la pupille du sujet. On réalise donc un balayage spécifique destiné à l'exploration de la microsurface proprement dite.

Dans ce mode de réalisation préféré, le flux d'illumination et le flux rétrodiffusé par les tissus empruntent le même trajet, de sorte que le faisceau de retour est stabilisé par le dispositif de déviation constitué par les deux miroirs 400 et 450. Ce flux de retour n'est pas recueilli dans le plan pupillaire comme dans le document US 4 213 678.

Le flux stabilisé et corrigé est transmis à un dispositif de filtration confocale ou de filtration spatiale, disposé dans le plan conjugué de la rétine, devant le détecteur destiné à imager la rétine.

Ce bloc de filtration spatiale confocal est constitué par un trou de filtration du diamètre d'une tache de diffraction. Il est situé, grâce à une lentille 600, dans un plan conjugué de la source 12 et du tissu rétinien exploré 5.

Le dispositif de réalisation d'image placé en aval de ce filtre confocal est constitué d'un détecteur 700 de type photomultiplicateur ou photodiode à avalanche ou CCD. Il détecte donc le flux retrodiffusé issu des tissus étudiés 5 et ayant traversé les éléments 500, 400, SM1, 450, 20 et 600. Le flux détecté par le capteur 700 est amplifié puis traité par le bloc électronique 42 pour afficher l'image tissulaire.

Le capteur 700 est ici unique, non matriciel. Le dispositif comporte donc un système électronique de conversion temporo-spatiale de l'information qui permet à partir d'un capteur unique de remplir une matrice de valeurs correspondant aux mesures effectuées en chaque point composant la microsurface.

L'information temporelle de flux rétrodiffusé est convertie en information spatiale par ce système électronique remplissant une matrice de façon synchrone et homothétique aux déplacements des systèmes de balayage 400 et 450.

Le dispositif à filtration confocale permet de travailler avec une bande spectrale large ou étroite. Les images de fluorescence sont également possibles.

Dans les conditions décrites ci-dessus la résolution est de 2 µm dans le plan de la surface rétinienne XY et de 10 µm dans la profondeur rétinienne Z. Un avantage important de ce dispositif est de permettre de faire des études en fluorescence. Un tel dispositif permet de réaliser des images large champ de plus de 10°.

Dans une seconde variante de l'invention, on réalise des images d'interférence entre un flux rétrodiffusé par le plan de tissu observé et un flux dans une branche de référence qui parcourt la même distance optique que le flux rétrodiffusé à la longueur de cohérence spatio-temporelle près.

La correction des déformations du front d'onde est effectuée sur le flux stabilisé avant la réalisation de l'addition cohérente avec le signal de la branche de référence.

La mesure du front d'onde est là encore effectuée par la formation d'un point d'illumination le plus petit possible dans le tissu exploré. Celui-ci est obtenu par une diode super radiante 11 et un dispositif optique apte à générer un point de concentration des rayons lumineux dans le tissu observé.

La source d'illumination 12 utilisée pour la réalisation d'image est de faible cohérence spatio-temporelle pour obtenir une résolution en Z de quelques micromètres. Il peut s'agir, par exemple, d'une diode électroluminescente. La résolution en Z dépend de la cohérence temporelle de la source d'illumination, et aussi de l'ouverture du système optique.

Le trajet effectué par les faisceaux lumineux dans la branche de référence et dans celle d'observation doivent donc être strictement identiques.

Pour réaliser ce but on dispose dans la branche de référence un dispositif opto-mécanique pouvant simuler au plus près le comportement optique de l'oeil. Lorsque les conditions de cohérence spatio-temporelles sont remplies pour les deux branches, il y a formation d'interférences qui après traitement informatique approprié (détection synchrone et calculs) permettent d'obtenir l'image du plan tissulaire étudié.

Le flux d'illumination fourni par la diode 12 est dirigé sur la rétine par l'intermédiaire d'un miroir 400 mobile apte à diriger ce flux sur une microsurface O1 isoplanétique choisie.

Le flux rétrodiffusé par la micro-surface étudiée O1 est dirigé vers une lame de mélange SM1.

Pour parvenir à cette lame de mélange SM1, le flux rétrodiffusé suit en sens inverse une partie du parcours du flux d'illumination. Il franchit notamment un miroir mobile de changement de microsurface 400, un miroir déformable 22 de compensation de front d'onde, un miroir de renvoi 23, un module de mesure de déformations de front d'onde 21 couplé au miroir 22, et arrive ensuite sur la lame de mélange SM1.

Le flux de référence est émis par la diode 11, dévié par le miroir séparateur semi-transparent SM1, puis parcourt le même trajet que le flux rétrodiffusé.

Le flux mélangé en sortie de SM1 est dirigé sur une seconde face du miroir 400, qui dévie ce flux correspondant à une surface isoplanétique entière sur un dispositif de réalisation d'image.

Le mélangeur SM1 assure donc le mélange du flux issu de la branche de référence constituée par les éléments précédemment décrits et référencés 11, SM1, SM2, 23, 22 et SM1 et du flux tissulaire rétro-diffusé issu des éléments précédemment décrits et référencés 5, 400, 22, 23, SM2, SM1.

Un dispositif optique en aval des diodes 11 et 12 et en amont du séparateur SM1 permet le mélange des flux sortants directement des diodes 11 et 12 ainsi que le réglage de la puissance du faisceau d'émission de chacune d'elles. Deux systèmes optiques obj2 et obj3 sont placés pour cela devant les diodes et permettent de choisir la position du plan étudié.

Les images d'interférence sont réalisées dans le plan d'un capteur 710 et correspondent, à chaque position du miroir 400, à l'ensemble de la surface isoplanétique que l'on désire visualiser.

La source 12 destinée à l'imagerie tissulaire forme une surface d'illumination sur la rétine d'un oeil 5 qui est égale, au grossissement optique près, à la surface de tissu correspondant à la micro-surface élémentaire étudiée.

Le grossissement optique fourni par les dispositifs optiques intermédiaires entre cette diode 12 et le tissu observé est calculé de façon à ce que l'ensemble de la micro-surface étudiée soit illuminé au même moment par la diode 12.

La configuration d'interférométrie est donc adaptée à réaliser des interférences sur la lumière provenant simultanément de toute une partie de l'oeil (ou d'un autre objet ou corps) de sorte qu'il réalise une image de cette partie par cette interférence d'ensemble, cette partie étant ici une surface isoplanétique.

Le dispositif de mesure d'image comprend une matrice de récepteurs, amplifiés ou refroidis, qui permettent de fournir une image électronique matricielle du tissu exploré.

Les interférences formées par l'interaction entre la branche de référence et la branche de mesure sont détectées par le capteur matriciel 710 puis traitées par un bloc électronique et informatique 42 pour extraire le signal et afficher l'image tissulaire.

Dans le cadre de cette variante de l'invention, on explore les microsurfaces isoplanétiques successivement grâce au dispositif de balayage 400 et également grâce à un cache dynamique 720 placé devant le capteur matriciel.

Le dispositif de cache dynamique 720 est constitué par un diaphragme balayant spatialement le détecteur plan de façon à couvrir successivement toute la surface de celui-ci.

En d'autres termes, le dispositif de filtration dynamique 720 découvre la plage du détecteur matriciel 710 correspondant à la micro-surface étudiée au grossissement près et la situe sur celui-ci. Le dispositif de filtration dynamique 720 et le détecteur matriciel 710 sont placés dans un plan conjugué optique du plan tissulaire étudié.

Par commutation successive en configuration transparente d'éléments électro-optiques de filtration, on constitue un masque correspondant à la surface d'analyse de tissu au grossissement optique près. Ce masque mobile balaye pas à pas le plan de la matrice destinée à réaliser l'image de l'ensemble des micro-surfaces. Les changements de position de l'ouverture sont synchrones et homothétiques des déplacements pas à pas du dispositif de balayage 400.

Le déplacement homothétique et synchrone de la position de la micro-surface sur la matrice photodétectrice 710 est assuré par la seconde face du miroir galvanométrique 400.

Avec une source de 633 nm, la résolution obtenue avec ce dispositif est de 2µm en XY et 10 µm en Z.

L'utilisation d'une longueur d'onde moyenne de l'ordre de 488 nm permet ainsi d'obtenir des résolutions de l'ordre du micrométre en X,Y et de quelques micromètres en Z pour l'exploration rétinienne.

Dans ce dispositif comme dans le cas du dispositif précédent, les moyens de compensation appliquent une correction à un ensemble de flux provenant d'un ensemble de points d'une surface choisie, ces flux ayant même déformation de front d'onde qu'un point de calcul de cette correction.

Dans le cas présent, les flux provenant de ces points sont prélevés simultanément de sorte qu'ils constituent un flux d'ensemble.

L'étendue de la surface de prélèvement est choisie pour que la correction mesurée au point de calcul soit valable pour l'ensemble de cette surface.

Le prélèvement de lumière rétrodiffusée est ici réalisé simultanément sur l'ensemble de la surface isoplanétique. On tire donc un bénéfice optimal de chaque nouvelle configuration du miroir de correction 22 en prélevant la lumière sur toute une surface, l'étendue de cette surface étant choisie pour que la compensation soit valable sur toute cette surface. Pour que le point de calcul de compensation soit bien situé dans la surface de prélèvement isoplanétique de prélèvement, la diode super-radiante 11 émet, dans une phase de mesure de déformation de front d'onde, un flux qui parcourt une partie de trajet qui est commune avec le trajet du flux d'illumination et de rétrodiffusion.

Dans l'exemple décrit ici, l'étendue des microsurfaces est prédéterminée par la disposition relative des différents éléments du dispositif. On prévoit toutefois de munir le dispositif d'un module de détermination automatique de l'étendue des surfaces apte à veiller automatiquement à leur isoplanétisme.

Dans le présent montage, les moyens de calcul de compensation (c'est à dire notamment le module de calcul 21 et le miroir 400 qui détermine le point de calcul) et les moyens de prélèvement d'image (c'est à dire les moyens d'interférométrie 11, SM1 et le miroir 400, sont synchronisés de sorte qu'à chaque nouvelle zone prélevée, un calcul de compensation adapté à cette zone est effectué.

Les montages selon l'invention décrits précédemment autorisent une compensation des amétropies du sujet. La compensation d'amétropie est réalisée soit en déplaçant conjointement le dispositif source et filtration confocale, pour le système à balayage confocal, soit en déplaçant le plan de référence pour le dispositif d'interférométrie.

Ces différents appareils peuvent être mis en oeuvre pour l'observation de la cornée, du cristallin et du fond d'oeil en temps réel. Les dispositions décrites précédemment permettent notamment de réaliser un biomicroscope ou un ophtalmoscope tridimensionnel ayant une résolution de l'ordre du micromètre.

Un autre avantage de l'invention est que le dispositif choisi autorise un fonctionnement en pupille d'illumination large, c'est-à-dire sans obligation de placer le sujet dans des conditions de vue Maxwellienne stricte.

L'invention améliore de façon très sensible, environ d'un facteur 10, la résolution selon les trois directions de l'espace tout en permettant d'explorer des champs importants.

Ces dispositifs fonctionnent pour toutes les longueurs d'onde pour lesquelles l'optique de l'oeil est transparente.

Cependant les dispositifs décrits ci-avant, qui font appel à une technique de balayage confocal ou à une technique d'interférométrie, ne se limitent pas à l'observation de l'oeil ou des corps vivants, ils sont avantageusement appliqués à l'observation d'objets manufacturés tels que des semi-conducteurs par exemple.

L'invention ne se limite pas non plus aux exemples de réalisation décrits précédemment.

On prévoit notamment selon l'invention de combiner les trois techniques : optique adaptative, balayage optique et interférométrie.

Le montage optique n'utilise pas nécessairement la règle de séparation des pupilles d'illumination et d'observation qui sont ici superposées ou confondues et ont le diamètre de la pupille du sujet.

Dans ces conditions, l'angle d'ouverture optique utilisable est plus grand, diminuant le diamètre de la tache de diffraction. La diminution de la tache de diffraction permet une augmentation additionnelle de la résolution.

Ceci permet d'utiliser une pupille optique plus grande que celle des appareils conventionnels, qui peut atteindre ici 7mm à 9 mm chez le sujet normal en mydriase. Cette disposition améliore encore la très bonne résolution de l'image.

## Revendications

1. Dispositif d'observation d'un corps, notamment d'un oeil, comportant :
- des moyens d'illumination (12) d'une surface dudit corps (5),
- des moyens pour prélever un flux lumineux rétrodiffusé depuis ladite surface illuminée,
- des moyens pour réaliser une image à partir du flux lumineux rétro-diffusé ainsi prélevé,
- des moyens de mesure de déformation de front d'onde, placés dans ledit flux lumineux rétrodiffusé, et
- des moyens de compensation de front d'onde, disposés en amont desdits moyens de mesure de déformation, agencés pour appliquer au flux lumineux prélevé une correction calculée à partir de mesures de déformation,
**caractérisé en ce qu'**il comprend en outre des moyens de balayage pour dévier le flux lumineux émis par les moyens d'illumination de façon à explorer une série de micro-surfaces selon un pas de balayage déterminé de sorte que les fronts d'onde émanant de tout point de chaque micro-surface explorée sont identiques à une fraction de longueur d'onde prés, et **en ce que** les moyens de réalisation d'image sont agencés pour fournir une exploration en profondeur Z de chaque micro-surface explorée.

2. Dispositif selon la revendication 1, **caractérisé en ce que** les moyens de réalisation d'image comprennent des moyens de filtration spatiale confocale disposés en amont des moyens de réalisation d'image et recevant le flux lumineux corrigé.

3. Dispositif selon la revendication 2, **caractérisé en ce que** les moyens de réalisation d'image comprennent des moyens pour réaliser une image d'interférence entre un flux rétro-diffusé depuis la surface illuminée et un flux dans une branche de référence qui parcourt la même distance optique que ledit flux rétro-diffusé à la longueur de cohérence spatio-temporelle près.

4. Dispositif selon la revendication 3, **caractérisé en ce que** les moyens d'illumination présentent une faible cohérence spatio-temporelle.

5. Dispositif selon l'une des revendications précédentes, **caractérisé en ce qu'**il comprend des moyens pour produire dans la zone de prélèvement un point de concentration de rayons lumineux d'éclairement et des moyens pour ne prélever que les rayons lumineux directs entre le point de concentration et une entrée optique des moyens de prélèvement.

6. Dispositif selon l'une des revendications 3 ou 4, **caractérisé en ce que** les moyens d'interférométrie (12, SM1, SM3) réalisent une image d'au moins une partie du corps (5) non réduite à un point par interférométrie sur simultanément la lumière provenant de l'ensemble de cette partie.

7. Dispositif selon l'une des revendications 3, 4 ou 6, **caractérisé en ce que** la compensation de front d'onde est appliquée au faisceau prélevé avant interférence de ce faisceau avec un faisceau de référence.

8. Dispositif selon l'une quelconque des revendications précédentes, **caractérisé en ce qu'**il comprend en outre un capteur matriciel et un cache modifiable synchronisé aux moyens de prélèvement pour découvrir à tout moment une plage du capteur matriciel correspondant à la zone de prélèvement en cours.

9. Dispositif selon la revendication 8, **caractérisé en ce que** le cache modifiable est constitué d'un ensemble d'éléments aptes à être commutés en position transparente ou en configuration opaque.

10. Dispositif selon l'une quelconque des revendications précédentes, **caractérisé en ce qu'**il est prévu pour balayer le point de concentration sur la zone de prélèvement.

11. Dispositif selon la revendication 10, **caractérisé en ce qu'**il comprend un premier module de déviation (450) apte à balayer le point de concentration dans la zone de prélèvement et un second module de déviation (400) apte à changer l'emplacement de la zone de prélèvement.

12. Dispositif selon l'une quelconque des revendications précédentes, **caractérisé en ce qu'**il comporte un capteur photométrique (700) apte à transformer la lumière prélevée en un signal de commande d'un dispositif de présentation d'image, et **en ce que** les moyens de mesure de déformation de front d'onde (21) et les moyens de correction (22) du front d'onde sont placés sur le trajet de la lumière entre le corps et le capteur (700).

13. Dispositif selon la revendication 12, **caractérisé en ce que** les moyens de correction de déformation du front d'onde (21) sont situés entre le capteur (700) et les modules de déviation (400, 450).

14. Dispositif selon l'une des revendications précédentes, **caractérisé en ce que** les moyens de filtration spatiale confocale comprennent un trou de filtration placé dans un plan conjugué de la zone de prélèvement, d'un diamètre d'une tache de filtration.

15. Dispositif selon l'une des revendications précédentes, **caractérisé en ce que** la zone de prélèvement (O1) est choisie suffisamment voisine du point de calcul de la compensation en cours pour que les fronts d'onde issus de tout point de cette zone (O1) soient identiques à un quart de longueur d'onde près.

16. Procédé pour observer un corps, notamment un oeil, comportant :
- une illumination d'une surface dudit corps par un flux lumineux,
- un prélèvement d'un flux lumineux rétro-diffusé depuis ladite surface illuminée,
- une mesure de déformation de front d'onde effectuée sur ledit flux lumineux rétro-diffusé,
- une réalisation d'une image à partir du flux lumineux rétro-diffusé ainsi prélevé, et
- une compensation de front d'onde effectuée à partir de mesures de déformation de front d'onde, dans laquelle on applique au flux lumineux prélevé une correction calculée à partir de mesures de déformation,
**caractérisé en ce qu'**il comprend en outre un balayage du flux lumineux de façon à explorer une série de micro-surfaces selon un pas de balayage déterminé de sorte que les fronts d'onde émanant de tout point de chaque micro-surface explorée sont identiques à une fraction de longueur d'onde près,
et **en ce que** la réalisation d'image est prévue pour fournir une exploration en profondeur Z de chaque micro-surface explorée.

17. Procédé selon la revendication 16, **caractérisé en ce que** la réalisation d'image comprend une filtration spatiale confocale du flux lumineux corrigé.

18. Procédé selon la revendication 16, **caractérisé en ce que** la réalisation d'image comprend une réalisation d'une image d'interférence entre un flux rétro-diffusé depuis la surface illuminée et un flux dans une branche de référence qui parcourt la même distance optique que ledit flux rétro-diffusé à la longueur de cohérence spatio-temporelle près.

19. Procédé d'observation selon l'une des revendications 16 à 18, **caractérisé en ce qu'**il comprend en outre une détermination automatique de l'étendue de chaque micro-surface explorée.

## Claims

1. Device for observing a body, especially an eye, comprising:
- means (12) for illuminating a surface of the said body (5),
- means for extracting a light flux backscattered from the said illuminated surface,
- means for forming an image from the backscattered light flux thus extracted,
- wavefront distortion measurement means placed in the said backscattered light flux and
- wavefront compensation means placed upstream of the said distortion measurement means, which are arranged to apply, to the extracted light flux, a correction calculated from distortion measurements,
**characterized in that** it furthermore includes scanning means for deflecting the light flux emitted by the illumination means so as to explore a series of microsurfaces with a scanning step defined so that the wavefronts emanating from any point on each explored microsurface are identical to within a fraction of a wavelength, and **in that** the image forming means are designed to provide an exploration in the depth Z of each explored microsurface.

2. Device according to Claim 1, **characterized in that** the image forming means comprise confocal spatial filtering means placed upstream of the image forming means and receiving the corrected light flux.

3. Device according to Claim 2, **characterized in that** the image forming means comprise means for forming an interference image between a flux backscattered from the illuminated surface and a flux in a reference arm which travels the same optical distance as the said backscattered flux to within the spatio-temporal coherence length.

4. Device according to Claim 3, **characterized in that** the illumination means have little spatio-temporal coherence.

5. Device according to one of preceding claims, **characterized in that** it comprises means for producing, in the sampling region, a point at which the illuminating light rays are focussed and means for sampling only the direct light rays between the focussing point and an optical entrance of the sampling means.

6. Device according to either of Claims 3 and 4, **characterized in that** the interferometric means (12, SM1, SM3) form an image of at least one portion of the body (5) not reduced to a point by interferometry simultaneously on the light coming from all of this portion.

7. Device according to one of Claims 3, 4 and 6, **characterized in that** the wavefront compensation is applied to the sampled beam before interference of this beam with a reference beam.

8. Device according to any one of the preceding claims, **characterized in that** it furthermore comprises a matrix sensor and a modifiable mask synchronized to the sampling means in order to expose, at any moment, an area of the matrix sensor corresponding to the region being sampled.

9. Device according to Claim 8, **characterized in that** the modifiable mask consists of a set of elements capable of being switched into a transparent position or into an opaque configuration.

10. Device according to any one of the preceding claims, **characterized in that** provision is made for the focussing point to be scanned over the sampling region.

11. Device according to Claim 10, **characterized in that** it comprises a first deflection module (450) capable of scanning the focussing point in the sampling region and a second deflection module (400) capable of changing the position of the sampling region.

12. Device according to any one of the preceding claims, **characterized in that** it includes a photometric sensor (700) capable of converting the sampled light into a signal for controlling an image presentation device and **in that** the wavefront distortion measurement means (21) and the wavefront correction means (22) are placed in the path of the light between the body and the sensor (700).

13. Device according to Claim 12, **characterized in that** the wavefront distortion correction means (21) are placed between the sensor (700) and the deflection modules (400, 450).

14. Device according to one of the preceding claims, **characterized in that** the confocal spatial filtering means comprise a filtering hole placed in a plane conjugate with the sampling region, with a diameter of a filtration spot.

15. Device according to one of the preceding claims, **characterized in that** the sampling region (O1) is chosen to be sufficiently close to the compensation calculation point in question so that the wavefronts coming from any point in this region (O1) are identical to within one quarter of the wavelength.

16. Method for observing a body, especially an eye, comprising:
- illumination of a surface of the said body with a light flux,
- extracting of a light flux backscattered from the said illuminated surface,
- wavefront distortion measurement carried out on the said backscattered light flux,
- formation of an image from the backscattered light flux thus extracted and
- wavefront compensation carried out on the basis of wavefront distortion measurements, in which compensation a correction calculated on the basis of distortion measurements is applied to the extracted light flux,
**characterized in that** it furthermore includes scanning of the light flux so as to explore a series of microsurfaces with a scanning step defined so that the wavefronts emanating from any point in each explored microsurface are identical to within a fraction of a wavelength,
and **in that** image formation is intended to provide an exploration in the depth Z of each explored microsurface.

17. Method according to claim 16, **characterized in that** the image formation comprises confocal spatial filtering of the corrected light flux.

18. Method according to Claim 16, **characterized in that** the image formation comprises formation of an interference image between a flux backscattered from the illuminated surface and a flux in a reference arm which travels the same optical distance as the said backscattered flux to within the spatio-temporal coherence length.

19. Method of observation according to one of Claims 16 to 18, **characterized in that** it furthermore includes automatic determination of the extent of each explored microsurface.

## Patentansprüche

1. Vorrichtung zur Beobachtung eines Körpers, insbesondere eines Auges, die folgendes umfaßt:
- Mittel zur Beleuchtung (12) einer Oberfläche des Körpers (5),
- Mittel zum Entnehmen eines von der beleuchteten Oberfläche rückgestreuten Lichtstromes,
- Mittel zum Erstellen eines Bildes ausgehend von dem so entnommenen rückgestreuten Lichtstromes,
- Mittel zum Messen der Deformation der Wellenfront, die im rückgestreuten Lichtstrom angeordnet sind, und
- Mittel zur Kompensation der Wellenfront, die stromauf der Mittel zum Messen der Deformation angeordnet und derart ausgestaltet sind, daß sie am entnommenen Lichtstrom eine Korrektur vornehmen, die ausgehend von Deformationsmessungen berechnet ist,
**dadurch gekennzeichnet, daß** sie ferner Abtastmittel zum Ablenken des von den Beleuchtungsmitteln ausgesandten Lichtstromes umfassen, um eine Abfolge von Mikrooberflächen gemäß einem Abtastschritt zu untersuchen, der derart bestimmt ist, daß die von allen Punkten jeder untersuchten Mikrooberfläche ausgehenden Wellenfronten auf einen Bruchteil der Wellenlänge genau identisch sind, und daß die Bilderstellungsmittel derart ausgestaltet sind, daß sie eine Z-Tiefen-Untersuchung jeder Mikrooberfläche liefern.

2. Vorrichtung nach Anspruch 1, **dadurch gekennzeichnet, daß** die Bilderstellungsmittel Mittel zum konfokalen räumlichen Filtern umfassen, die stromauf der Bilderstellungsmittel angeordnet sind und den korrigierten Lichtstrom empfangen.

3. Vorrichtung nach Anspruch 2, **dadurch gekennzeichnet, daß** die Bilderstellungsmittel Mittel zum Erstellen eines Interferenzbildes zwischen einem von der beleuchteten Oberfläche rückgestreuten Strom und einem Strom in einem Referenzzweig umfaßt, der auf eine räumlich-zeitliche Kohärenzlänge genau die gleiche optische Distanz wie der rückgestreute Strom durchläuft.

4. Vorrichtung nach Anspruch 3, **dadurch gekennzeichnet, daß** die Beleuchtungsmittel eine schwache räumlich-zeitliche Kohärenz aufweisen.

5. Vorrichtung nach einem der vorhergehenden Ansprüche, **dadurch gekennzeichnet, daß** sie Mittel zum Erzeugen in der Entnahmezone eines Konzentrationspunktes der Belichtungs-Lichtstrahlen und Mittel nur zum Entnehmen direkter Lichtstrahlen zwischen dem Konzentrationspunkt und einem optischen Eingang der Entnahmemittel umfaßt.

6. Vorrichtung nach einem der Ansprüche 3 oder 4, **dadurch gekennzeichnet, daß** die Interferometriemittel (12, SM1, SM3) ein Bild wenigstens eines nicht auf einen Punkt reduzierten Teils des Körpers (5) durch Interferometrie über gleichzeitig des von der Gesamtheit dieses Teils stammenden Lichtes erstellen.

7. Vorrichtung nach einem der Ansprüche 3, 4 oder 6, **dadurch gekennzeichnet, daß** die Kompensation der Wellenfront am entnommenen Strahl vor Interferenz dieses Strahls mit einem Referenzstrahl vorgenommen wird.

8. Vorrichtung nach einem der vorhergehenden Ansprüche, **dadurch gekennzeichnet, daß** sie ferner einen Matrixsensor und eine modifizierbare Maske umfaßt, die mit den Entnahmemitteln synchronisiert ist, um zu jedem Zeitpunkt einen Bereich des Matrixsensors freizulegen, der der laufenden Entnahmezone entspricht.

9. Vorrichtung nach Anspruch 8, **dadurch gekennzeichnet, daß** die modifizierbare Maske aus einer Gesamtheit von Elementen zusammengesetzt ist, die derart ausgestaltet sind, daß sie in eine durchsichtige Position oder eine undurchsichtige Konfiguration geschaltet werden können.

10. Vorrichtung nach einem der vorhergehenden Ansprüche, **dadurch gekennzeichnet, daß** sie zum Abtasten des Konzentrationspunktes über der Entnahmezone vorgesehen ist.

11. Vorrichtung nach Anspruch 10, **dadurch gekennzeichnet, daß** sie ein erstes Ablenkmodul (450) umfaßt, das derart ausgestaltet ist, daß es den Konzentrationspunkt in der Entnahmezone durchfährt, und ein zweites Ablenkmodul (400), das derart ausgestaltet ist, daß es den Ort der Entnahmezone ändert.

12. Vorrichtung nach einem der vorhergehenden Ansprüche, **dadurch gekennzeichnet, daß** sie einen photometrischen Sensor (700) umfaßt, der derart ausgestaltet ist, daß er das entnommene Licht in ein Steuersignal einer Vorrichtung zum Darstellen eines Bildes umwandelt, und daß die Mittel zum Messen der Deformation der Wellenfront (21) und die Mittel zur Korrektur (22) der Wellenfront auf dem Lichtpfad zwischen dem Körper und dem Sensor (700) angeordnet sind.

13. Vorrichtung nach Anspruch 12, daß die Mittel zur Korrektur der Deformation der Wellenfront (21) zwischen dem Sensor (700) und den Ablenkmodulen (400, 450) liegen.

14. Vorrichtung nach einem der vorhergehenden Ansprüche, **dadurch gekennzeichnet, daß** die Mittel zum konfokalen räumlichen Filtern ein Filterloch umfassen, das in einer zur Entnahmezone zugeordneten Ebene angeordnet ist, mit einem Durchmesser eines Filterfleckes.

15. Vorrichtung nach einem der vorhergehenden Ansprüche, **dadurch gekennzeichnet, daß** die Entnahmezone (O1) so gewählt ist, daß sie genügend nah am laufenden Kompensationsberechnungspunkt liegt, damit die von allen Punkten dieser Zone (O1) ausgesandten Wellenfronten auf ein Viertel der Wellenlänge genau identisch sind.

16. Verfahren zum Beobachten eines Körpers, insbesondere eines Auges, das folgende Schritte umfaßt:
- eine Beleuchtung der Oberfläche mit einem Lichtstrom,
- eine Entnahme eines von der beleuchteten Oberfläche rückgestreuten Lichtstromes,
- eine Messung der Deformation der Wellenfront, die am rückgestreuten Lichtstrom hervorgerufen wird,
- ein Erstellen eines Bildes ausgehend von dem so entnommenen rückgestreuten Lichtstrom, und
- eine Kompensation der Wellenfront, die ausgehend von Messungen der Deformation der Wellenfront bewirkt wird, bei der am entnommenen Lichtstrom eine Korrektur vorgenommen wird, die ausgehend von Messungen der Deformation berechnet wird,
**dadurch gekennzeichnet, daß** es ferner ein Abtasten des Lichtstroms umfaßt, um eine Abfolge von Mikrooberflächen gemäß einem Abtastschritt zu untersuchen, der derart bestimmt ist, daß die von allen Punkten jeder untersuchten Mikrooberfläche ausgehenden Wellenfronten auf einen Bruchteil der Wellenlänge genau identisch sind,
und daß die Erstellung eines Bildes vorgesehen ist, um eine Z-Tiefen-Untersuchung jeder untersuchten Mikrooberfläche zu liefern.

17. Verfahren nach Anspruch 16, **dadurch gekennzeichnet, daß** die Erstellung eines Bildes ein konfokales räumliches Filtern des korrigierten Lichtstromes umfaßt.

18. Verfahren nach Anspruch 16, **dadurch gekennzeichnet, daß** die Erstellung eines Bildes eine Erstellung eines Interferenzbildes zwischen einem von der beleuchteten Oberfläche rückgestreuten Stroms und eines Stroms in einem Referenzzweig umfaßt, der auf eine räumlichzeitliche Kohärenzlänge genau die gleiche optische Distanz wie der rückgestreute Strom durchläuft.

19. Beobachtungsverfahren nach einem der Ansprüche 16 bis 18, **dadurch gekennzeichnet, daß** es ferner eine automatische Bestimmung der Ausdehnung jeder untersuchten Mikrooberfläche umfaßt.
